# EUROPEAN PATENT APPLICATION

(11) **EP 4 717 769 A1**
(43) Date of publication of application: **01.04.2026**
(21) Application number: 25204809.5
(22) Date of filing: 26.09.2025
(51) Int. Cl.: C12N 15/10

(54) **METHOD FOR ISOLATING NUCLEIC ACIDS**

(30) Priority: 26.09.2024 EP 24202958
(71) Applicant: Xpedite Diagnostics GmbH, 85399 Hallbergmoos (DE)
(72) Inventor: Wende, Andy, 81477 München (DE); Gjika, Ejona, 82110 Germering (DE)
(74) Representative: Rückerl, Florian

(57) **Abstract**

The present invention relates to a method for isolating nucleic acids by providing a sample with nucleic acids, a buffer and soluble albumin, precipitating the albumin and separating the precipitated albumin from the supernatant of the sample. The present invention also relates to compositions and kits for carrying out such method.

## Description

The present invention relates to a method for isolating nucleic acids by providing a sample with nucleic acids, a buffer and soluble albumin, precipitating the albumin and separating the precipitated albumin from the supernatant of the sample. The present invention also relates to compositions and kits for carrying out such method.

For many biotechnological applications, and in particular in the context of molecular diagnostics, nucleic acids need to be isolated and concentrated. For this purpose, the standard method in the art is the "bind-wash-elute" approach, which has been established about 30 years ago. Said approach relies on the binding of DNA or RNA, respectively to silica coated surfaces in presence of chaotropic salts and alcohols (Boom method). By this means nucleic acids can be isolated and concentrated form essentially any sample. However, the method relies on large amounts of substances harmful to the environment such as guanidinium salts and alcohols (e.g. ethanol and isopropanol). Guanidine is typically produced via chemical synthesis, which requires necessarily energy. In view of the growing demand for nucleic acid extractions, the impact on the environment may not be underestimated. For instance, in the context of the COVID-19 pandemic alone, much more than one billion RNA extractions where carried out around the globe. Other nucleic acid extraction methods, which are less common, rely on binding of nucleic acids to carboxy modified surfaces in presence of high concentrations of polyethylene glycol (PEG) and sodium chloride. A further approach in the art relies on sample extraction by means of direct lysis. The nucleic acids are extracted here by using a heat step, but these methods typically do not allow to concentrate the nucleic acids or to remove inhibitors or other contaminants from the lysate. The utility of the latter methods is thus limited.

Therefore, there is a clear need in the art for alternative methods for isolating and concentrating nucleic acids, which ideally avoid the above-mentioned disadvantages of the art, such as the use of substances harmful to the environment.

This problem is solved by the subject-matter as set forth in the appended claims and in the description below.

As will be shown in the following, the inventors of the present invention have surprisingly found that albumin proteins, which are environmentally harmless, can be used to isolate nucleic acids from a sample solution very efficiently. If albumin is added to the sample containing the nucleic acids of interest, said albumin will interact with the nucleic acids, presumably via electrostatic interactions. If the albumin is then precipitated from the sample, the nucleic acids will be depleted from the sample solution in parallel. Thereafter, the precipitate can be separated from the supernatant of the sample and the nucleic acids can be eluted from the albumin precipitate. This entire process is very efficient and does not require any harmful substances. The nucleic acids obtained in such manner can be used for other biotechnological methods, such as sequencing of the nucleic acids.

Therefore, the present invention relates in a first aspect to a method for isolating nucleic acids from a sample, the method comprising the following steps: a) providing a sample comprising nucleic acids, soluble albumin and optionally a buffer; b) precipitating the albumin in the sample and c) separating the precipitated albumin (and in parallel nucleic acids adhering thereto) from the supernatant of the sample.

In a second aspect, the present invention relates to a composition or kit comprising i) a buffer suitable for buffering a sample at a pH of 6.5 or lower, preferably in the range of 3.5 to 6.5, preferably at a pH of 5, b) soluble albumin and c) magnetic or non-magnetic particles.

The method of the invention requires the provision of a sample comprising nucleic acids, soluble albumin and optionally a buffer. The buffer is selected to ensure in the sample an acidic pH of 6.5 or lower, preferably in the range of 3.5 to 6.5. The pH may be 3.5., 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4 or 6.5. Preferably, the pH is in the range of 4.0 to 6.0, even more preferably in the range of 4.5 to 5.5. Typically, the pH of the sample will be in the range of - 1.2 pH to +1.2 pH compared to the isoelectric point (IEP) of the albumin in an attempt to minimize the net electrical charge of the albumin. Preferably the pH is in the range of - 0.5 pH to + 0.5 pH of the IEP. The actual type of buffer is not of particular significance provided it buffers at a pH in the indicated ranges. The buffer may for example be a mixture comprising Tris buffer, acetate buffer and phosphate buffer (TAP buffer), or be an acetic acid, citric acid, lactic acid, malic acid or phosphoric acid buffer. A preferred buffer according to the present invention is the TAP buffer, but essentially all of the aforementioned buffers achieve similar results. The buffer may also comprise salt.

The soluble albumin is preferably a naturally occurring albumin without any synthetic chemical modifications. In particular, the albumin is preferably an albumin which has not been synthetically methylated or esterified. Preferably, the albumin is a serum albumin. Serum albumins are specific albumins which are present in the blood stream of vertebrates. Even more preferably, the albumin is a mammalian serum albumin such as bovine serum albumin (BSA) or human serum albumin (HSA). In some embodiments, the albumin will be an albumin other than a serum albumin. Such other albumins suitable for carrying out the invention include - without being limited thereto - ovalbumin (OVA), which is present in egg white, and lactalbumin (LA), which is the albumin contained in the milk of many mammals. In the context of the present invention, BSA is a particularly preferred albumin, as it is well studied and abundantly available. Typically, the sample provided in step a) of the inventive method will comprise only one type of albumin, such as BSA. However, the inventors also conceive that mixtures of two or more albumins may be used, i.e. the requirement that the sample comprises a soluble albumin does not imply that only one type of albumin may be present. Moreover, the albumin is "soluble" albumin. As used herein, "soluble albumin" refers to the free protein, i.e. it is not immobilized on or crosslinked to any type of surfaces or particles. Moreover, the protein needs to be "soluble", i.e. can be dissolved in a liquid, aqueous sample. In embodiments where the albumin of choice is BSA; the pH of the sample is preferably in the range of 4.2 to 6.0, preferably in the range of 4.5 to 5.5, and is most preferably 5.0. In embodiments, where the albumin of choice is OVA, the pH of the sample is preferably in the range of 3.8 to 6.0, preferably in the range of 4.0 to 5.5, and is most preferably 5.0. In embodiments, where the albumin of choice is LA, the pH of the sample is preferably in the range of 3.5 to 6.0, more preferably in the range of 3.5 to 5.0, more preferably in the range of 3.5 to 4.5, and is most preferably 4.0. The concentration of the soluble albumin, such as BSA, in the sample of step a) of the inventive method is preferably in the range of 0.05 to 1.0 % (w/v), more preferably in the range of 0.05 to 0.5 % (w/v) and is most preferably 0.5 % (w/v).

The sample provided in step a) of the inventive method will in many cases be - without being limited thereto - a biological sample (i.e. of biological origin) obtained previously and containing nucleic acids, to which the buffer and soluble albumin have been added. Thus, the sample of step a) will be preferably a liquid, aqueous solution or suspension. The type of biological sample is not limited by the present invention. The biological sample may for example be blood (e.g. human blood), in particular blood plasma (e.g. human blood plasma), cerebrospinal fluid, in particular human cerebrospinal fluid, faeces, in particular human faeces, or a biopsy sample from a mammal such as a human. It needs to be noted that under certain circumstances no additional albumin needs to be added. Where the biological sample already comprises albumin naturally, i.e. no additional albumin will be added. In some embodiments, formally non-biological samples (i.e. not directly of biological origin in the strict sense but comprising or suspected of comprising as biological component nucleic acids) may be combined with the buffer and the soluble albumin. For example, in the context of the present invention a sample may be an aqueous sample comprising or suspected of comprising nucleic acids (for instance contained in a microorganism in the sample). The aqueous sample may be for example a sample taken from the sea, from a freshwater source such as a spring, lake, or river, tap water, water provided by public water suppliers, wastewater, sewage etc. The sample may also be an aqueous suspension of solid samples such as an earth sample suspended in a water or buffer. Such earth sample will typically comprise microorganisms and thus contain nucleic acids. In some cases, the sample may have been pre-treated to render the nucleic acids more readily accessible. This is particularly recommendable in situations where the nucleic acids of the biological samples are initially still contained in microorganisms, cells or tissue. Preferably, the sample of step a) comprises NaCl in a concentration of no more than 100 mM. In some embodiments of the invention, step a) of the inventive method of the first aspect of the invention may be preceded by the step of adding a buffer and/or soluble albumin to a sample comprising nucleic acids, such as a biological or non-biological sample as defined above.

The nucleic acids of the sample provided in step a) of the inventive method can be DNA, RNA or mixtures of DNA and RNA. The nucleic acids of the sample may comprise for example human DNA, human RNA or a mixture of human DNA and human RNA. The plural used in the term "nucleic acids" is not intended to imply that the sample needs to comprise different types of nucleic acids. Instead, the invention will also work in scenarios where multiple copies of one and the same nucleic acid are present in the sample. However, in many scenarios the sample will be derived from a biological sample which typically comprises in turn a plethora of different nucleic acids, and will include DNA as well as RNA. Therefore, it is particularly preferred in the present invention that the sample comprises DNA as well as RNA. The nucleic acids may be for example of eukaryotic, prokaryotic or viral origin or be a mixture thereof. In preferred examples of the invention, the sample comprises genomic DNA, in particular genomic DNA of human origin. In other scenarios, the DNA may for example be plasmid DNA. Where the sample comprises RNA, said RNA may be single stranded or double stranded. The RNA may be for instance rRNA or mRNA. Preferably, samples comprising DNA comprise DNA with a size in the range of 0.2 to 100 kb. Preferably, samples comprising RNA comprise RNA with a size in the range of 500 to 3000 nucleotides. Preferably, the RNA is rRNA.

Aside of the nucleic acids, the soluble albumin and the buffer, the sample may comprise optionally additional components. In a particularly preferred embodiment, the sample will comprise magnetic or non-magnetic particles which can aid in separating the precipitated albumin from the rest of the sample. The person skilled in the art is readily familiar with the concept of using magnetic or non-magnetic particles in biotechnological applications, in particular in those where separation is the objective. Such particles or beads are typically synthetic products with a size in the range of 100 nm to 10 µm and with a magnetic or non-magnetic core, and are frequently further functionalized with various biomolecules such as antibodies, antigens, proteins, catalyzers, or nucleic acids. In the present case, these particles (or beads) are preferably not functionalized with further biomolecules, because such function is not required for the purposes of the present invention. In embodiments, where the sample of step a) of the present invention comprises additionally magnetic or non-magnetic particles, said particles will interact with the precipitate obtained in step b) when precipitating the albumin, giving the precipitate a more compact form and stability. This in turn will facilitate separation of the precipitate from the supernatant, in particular if magnetic particles are used, which is more preferred than using non-magnetic participles. Such magnetic or non-magnetic particles can be added for example to a biological sample prior, concomitant or after the albumin and/or buffer has been added to said biological sample to provide the sample of step a). In some embodiments of the invention, a proportion but not all of the soluble albumin in the sample of step a) will directly adsorb to the magnetic or non-magnetic particles. For the purposes of the present invention, suitable non-magnetic participles may for instance be selected from kaolinite, polyvinylpolypyrrolidone (PVPP), or silica-based particles such as diatomaceous earth (e.g. Celite^{®} 545). Magnetic particles may for example also be silica particles, polystyrene particles or carboxylated particles (all in this case with magnetic core). Preferably, the magnetic particles are carboxylated particles.

Aside of magnetic or non-magnetic solid particles in the sample of step a), which assist separation in physical terms, the sample of step a) may also comprise (instead of or in addition to the particles) compounds supporting the later precipitation of the soluble albumin. The inventors of the present invention consider in this regard in particular polyethylenglycol, such as PEG 6000 (e.g. from Sigma-Aldrich), polyvinyl pyrrolidone, in particular PVP-10 (e.g. from Sigma-Aldrich), and ethoxylated nonylphenol, in particular NP-40, or IGEPAL^{®} CA-630 (e.g. from Sigma-Aldrich), which is octylphenoxy poly(ethyleneoxy)ethanol, to be suitable additives for this purpose. Therefore, in some embodiments of the invention, the sample of step a) of the inventive method will comprise one or more compounds selected from the group consisting of polyethylene glycol, polyvinylpyrrolidone and ethoxylated nonylphenol. In some further embodiments of the invention, the sample of step a) of the inventive method will comprise a polysorbate detergent like Tween^{®} 20, preferably in a concentration of no more than 1% (w/v). Such polysorbate detergent, just like octylphenoxy poly(ethyleneoxy)ethanol, will enhance elution results in an optional elution step of the inventive method. However, for optimal precipitation results it is preferred if the sample does not comprise any of the compounds listed in the following group: sodium dodecyl sulphate, cetyltrimethylammonium bromide, polysorbate, sorbitol and hydroxyproline.

Step b) of the method of the present invention requires that the soluble albumin in the sample of step a) is precipitated. By precipitation of the previously soluble albumin, nucleic acids will also be depleted from the supernatant because they adhere to the albumin protein. According to the present invention, this is preferably accomplished by using heat, which will precipitate the albumin very efficiently. The precipitation rate will correlate with the temperature employed and the time of incubation at said temperature. The lower the temperature, the longer it will take for the precipitate to form. In case of the present invention, precipitating in step b) involves incubating the sample preferably at a temperature of at least 65°C, more preferably at a temperature of at least 70°C, more preferably at a temperature of at a temperature of at least 75°C, more preferably at a temperature of at a temperature of at least 80°C, more preferably at a temperature of at a temperature of at least 85°C, even more preferably at a temperature of at least 95°C. The temperature in step b) may for example be in the range of 65°C to 98°C, more preferably in the range of 80°C to 95°C. The incubation time at the elevated temperatures in step b) needs to be sufficient to precipitate the soluble albumin, which will in most cases take a few minutes. Preferably, the time period will be in the range of 2 minutes to 20 minutes, preferably in the range of 5 minutes to 15 minutes. As mentioned above, the time period can be shorter at higher temperatures than at lower temperatures. Appropriate incubation times can be easily established by the skilled person, for example by visual control of the sample, as precipitated albumin can readily be detected in the sample. In a preferred embodiment, the sample is incubated at least 90°C for at least 5 minutes.

The present invention relies on different means to separate nucleic acids from a sample than the conventional Boom methods do. Therefore, it is particularly preferred that the sample is at the stage of precipitating the albumin from the sample (i.e. step b) essentially free of chaotropic salts and/or alcohols, i.e. the compounds utilized in the Boom based methods. For instance, the sample in step b) may be essentially free of guanidinium salts, ethanol and/or isopropanol. Preferably, the sample in step b) is essentially free of or is free of all of these compounds. "Essentially free of", as used herein, implies that no chaotropic salts and/or alcohols have been added to a biological sample in order to provide the sample of step a), but does not rule out that such salts or alcohols are already present naturally in a biological sample forming the basis for the sample of step a).

It is readily conceivable that the precipitate may also comprise other components, depending on the composition of the sample of step a). The sample may for instance comprise other proteins than albumin from the original biological sample. However, these are components which are irrelevant for carrying out the present invention. If herein reference is made to a precipitate, or albumin precipitate, this is not intended to specify a grade of purity. However, the precipitate formed in step b) of the inventive method will comprise at least the albumin and nucleic acids which adhere to said precipitated albumin. In some embodiments, the precipitate may additionally comprise magnetic or non-magnetic particles. Moreover, it is understood that step b) does not require precipitating the albumin quantitatively so that no soluble albumins will remain. However, by incubating the albumin at high temperatures as indicated above, usually the majority of albumin will precipitate.

After precipitation, the method will comprise the following additional step: c) separating the precipitated albumin (also referred herein as "the precipitate") from the supernatant of the sample. This can be done for example by simply discarding the supernatant while retaining the precipitated albumin. The person skilled in the art will be readily familiar with techniques of how to separate a supernatant from a solid component like the precipitate in the present case. Centrifuging the sample is frequently helpful to facilitate separation, because the precipitate may in some cases not readily settle down in its own. If solid phase magnetic or non-magnetic particles have been added to the sample of step a), then such particles will also be helpful in this step c) of the inventive method. The precipitate will at least partially adhere to these particles, which in turn can be easily centrifuged. In the case of magnetic particles, separation of precipitate and supernatant can even be further supported by using a magnet (magnetic separation). In this context it is noted that the inventors have also verified that the aforementioned magnetic or non-magnetic particles can also be added after precipitation, i.e. they need to be present already in the sample of step a) of the method according to the present invention. Therefore, in some embodiments, the method according to the present invention will involve after step b) (and before step c) the following additional step b'): mixing the albumin precipitated in step b) with magnetic or non-magnetic particles. As regard the type and nature of the magnetic or non-magnetic particles, reference is made to the discussion of these particles in the context of step a) of the inventive method.

Additionally, the method according to the present invention may comprise the step of: d) eluting the nucleic acids from the precipitated albumin by incubating the precipitated albumin in an elution buffer. For elution, the elution buffer needs to have a pH which is higher (more basic) than the buffer used in the sample of step a). Preferably, the elution buffer has a pH in the range of 8 to 12, more preferably in the range of 10 to 12. When selecting the pH of the elution buffer, the nature of the nucleic acid to be eluted is of importance. While DNA can be eluted at any pH between 8 and 12, and is preferably eluted at a pH of 12, RNA is instable at high pH such as pH 12 and should thus be preferentially eluted at lower pH values such as pH 10. Elution may be done for example in a range of 20°C - 98°C. Preferably, elution is done at temperatures above room temperature. Preferably, the precipitated albumin is incubated in the elution buffer at a temperature in the range of 80°C to 98°C. More preferably, the precipitated albumin is incubated in the elution buffer at a temperature in the range of 85°C to 95°C and even more preferably at a temperature in the range of 90°C to 95°C. Incubation in the elution buffer will usually not exceed a few minutes and is preferably in the range of 1 min to 10 minutes, such as 1, 2, 3, 4, 5, 6, 7, 8, 9 or 10 minutes, and is more preferably in the range of 1 min to 5 minutes and is most preferably 5 minutes. DNA is most preferably eluted by incubating the precipitated albumin in an elution buffer with pH 12 at 95°C for about 5 minutes. RNA is most preferably eluted by incubating the precipitated albumin in an elution buffer with a pH in the range of 8 to 10 for about 5 minutes.

The nature of the elution buffer is not critical for carrying out the invention. Preferably, the elution buffer is a TAP buffer adjusted to the desired pH, such as pH 12.

The elution buffer may additionally comprise a protease. Such protease will support elution of nucleic acids from precipitated albumin such as BSA. In a particularly preferred embodiment of the invention the protease is proteinase K. Other suitable proteases include, without being limited thereto, subtilisin, papain, ficin, pronase, and keratinase. The presence of a protease such as proteinase K is in particular recommendable where RNA is to be eluted from the precipitated protein. In such scenarios, the elution buffer preferably provides for a pH in the range of 8 to 10 and the elution buffer additionally comprises a protease such as proteinase K.

Finally, the method according to present invention may comprise the additional step of: e) separating the eluted nucleic acids from the precipitated albumin. Essentially, said final step may be carried out in analogous manner to step c), except of the fact that this time the supernatant is retained and the precipitate discarded. Step e) may also benefit from using solid particles, which may be magnetic or non-magnetic. Said particles are preferably already present from one of the preceding steps of the invention. However, it would in principle also be possible to add such particles for the first time or additionally when carrying out step e) of the inventive method.

Preferably, the method according to the first aspect of the invention comprises the following steps: a) mixing a biological or non-biological sample comprising nucleic acids with bovine serum albumin, a buffer providing for a pH of 6.5 or lower, preferably in the range of 3.5 to 6.5, and magnetic particles, b) precipitate the bovine serum albumin by heating to 90°C for 5 minutes, c) separating the precipitated bovine serum albumin from the supernatant of the sample by immobilizing the magnetic particles with a magnet and discarding the supernatant, and d) eluting the nucleic acids in an elution buffer at pH 12 for 5 minutes.

As mentioned above, the present invention relates in a second aspect to a composition or kit of parts comprising i) a buffer suitable for buffering a sample at a pH of 6.5 or lower, preferably in the range of 3.5 to 6.5, more preferably at a pH of 5, b) soluble albumin and c) magnetic or non-magnetic particles. Such composition or kit will be useful for carrying out the inventive method according to the first aspect of the invention. The buffer suitable for buffering a sample at a pH in the range of 3.5 to 6.5 may be selected to ensure in a sample an acidic pH at 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4 or 6.5. Preferably, the pH is in the range of 4.0 to 6.0, even more preferably in the range of 4.5 to 5.5. Typically, the buffer will provide for a pH in the range of - 1.2 pH to +1.2 pH compared to the isoelectric point (IEP) of the albumin of the kit or composition in an attempt to minimize the net electrical charge of the albumin. Preferably the pH is in the range of - 0.5 pH to + 0.5 pH of the IEP. The actual type of buffer is not of particular significance provided it buffers in the indicated ranges. The buffer may for example be a TAP buffer, an acetic acid, citric acid, lactic acid, malic acid or phosphoric acid buffer. A preferred buffer according to the present invention is the TAP buffer, but essentially all of the aforementioned buffers achieve similar results.

The soluble albumin of the composition or kit is preferably a naturally occurring albumin without any synthetic chemical modifications. In particular, the albumin is preferably an albumin which has not been synthetically methylated or esterified. Even more preferably, the albumin is a mammalian serum albumin such as bovine serum albumin (BSA) or human serum albumin (HSA). Other suitable albumins include ovalbumin (OVA) and lactalbumin (LA). In the context of the present invention, BSA is a particularly preferred albumin. Typically, the composition or kit of the invention will comprise only one type of albumin, such as BSA. However, the inventors also conceive that two or more albumins may be present in the composition or kit of the invention. Moreover, the albumin is "soluble" albumin. Moreover, the protein needs to be "soluble", i.e. can be dissolved in a liquid, aqueous sample. The composition or kit according to the present invention may comprise the albumin in lyophilized form or already dissolved in aqueous solution. In embodiments where the albumin of choice is BSA; the buffer of the composition or kit provides preferably for a pH in the range of 4.2 to 6.0, preferably in the range of 4.5 to 5.5, and provides most preferably for a pH of 5.0. In embodiments, where the albumin of choice is OVA, the buffer of the composition or kit provides preferably for a pH in the range of 3.8 to 6.0, preferably in the range of 4.0 to 5.5, and provides most preferably for a pH of 5.0. In embodiments, where the albumin of choice is LA, the buffer of the composition or kit provides preferably for a pH in the range of 3.5 to 6.0, preferably in the range of 3.5 to 4.5, and provides most preferably for a pH of 4.0.

The composition or kit of the invention may further comprise magnetic or non-magnetic particles, which are preferably not functionalized with biomolecules. For the purposes of the present invention, suitable non-magnetic participles may for instance be selected from kaolinite, polyvinylpolypyrrolidone (PVPP), or silica based particles such as Celite^{®} 545. Magnetic particles may for example also be silica particles, polystyrene particles or carboxylated beads (all in this case with magnetic core). Preferably, the magnetic particles are carboxylated.

The composition of kit of the present invention may also comprise (instead of or in addition to the particles) compounds supporting the later precipitation of the soluble albumin, in particular polyethylenglycol, such as PEG 6000 (e.g. from Sigma-Aldrich), polyvinyl pyrrolidone, in particular PVP-10 (e.g. from Sigma-Aldrich), ethoxylated nonylphenol, in particular NP-40 (e.g. from Sigma-Aldrich) or octylphenoxy poly(ethyleneoxy)ethanol (e.g. IGEPAL^{®} CA-630 from Sigma-Aldrich). Therefore, in some embodiments of the invention, the composition or kit of the invention will comprise one or more compounds selected from the group consisting of polyethylene glycol, polyvinylpyrrolidone and ethoxylated nonylphenol. In further embodiments of the invention, the composition or kit according to the present invention will comprise a polysorbate detergent like Tween^{®} 20.

The kit according to the present invention may also comprise magnetic means which allow for separation of magnetic particles from a sample.

Finally, and in a particularly preferred embodiment, the kit according to the present invention additionally comprises an elution buffer allowing elution of nucleic acids from precipitated albumin. Preferably, the elution buffer provides for a pH in the range of 8 to 12, more preferably in the range of 10 to 12. The elution buffer may be a TAP buffer, e.g. comprising 50 mM Tris, 50 mM acetate, and 50 mM phosphate, and optionally but preferably NaCl, e.g. 25 mM NaCl.

Furthermore, the kit of the present invention may additionally comprise a protease like proteinase K. Other suitable proteases include, without being limited thereto, subtilisin, papain, ficin, pronase, and keratinase.

A kit of the present may in particular comprise a) a buffer providing for a pH of pH or lower, preferably in the range of 3.5 to 6.5, b) bovine serum albumin, c) magnetic particles and d) and elution buffer providing for pH 12.

The term "comprising", as used herein, shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

### Figures

In the following a brief description of the appended figures will be given. The figures are intended to illustrate aspects of the present invention in more detail. However, they are not intended to limit the overall scope of the invention.

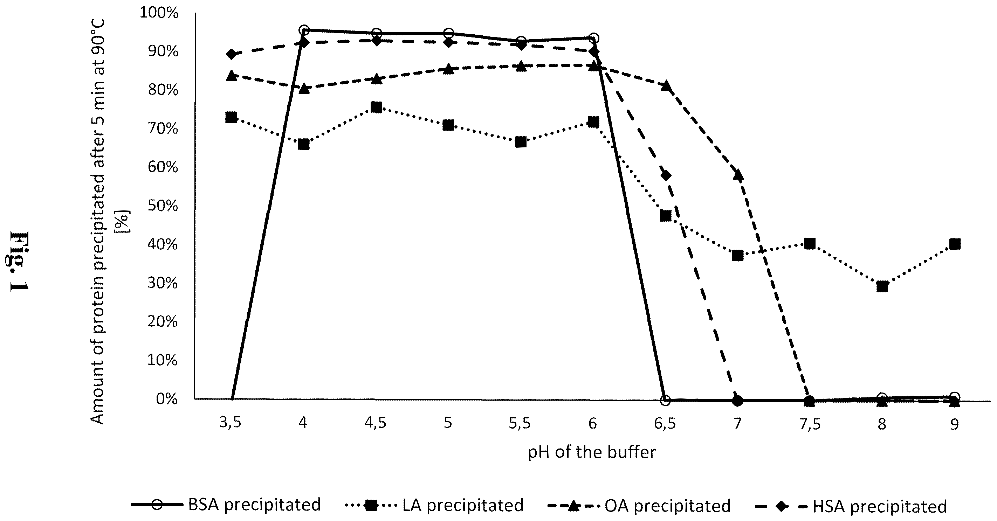

Fig. 1 illustrates the precipitation characteristics for bovine serum albumin (BSA), lactalbumin (LA), ovalbumin (OA) and human serum albumin (HSA) after 5 minutes of incubation at 90°C in TAP buffer. All four types of albumin precipitate at acidic pH values below 6.5. The amount of precipitation was determined by measuring the absorbance at 280nm (A280) of the initial samples as well as of the supernatant after precipitation.

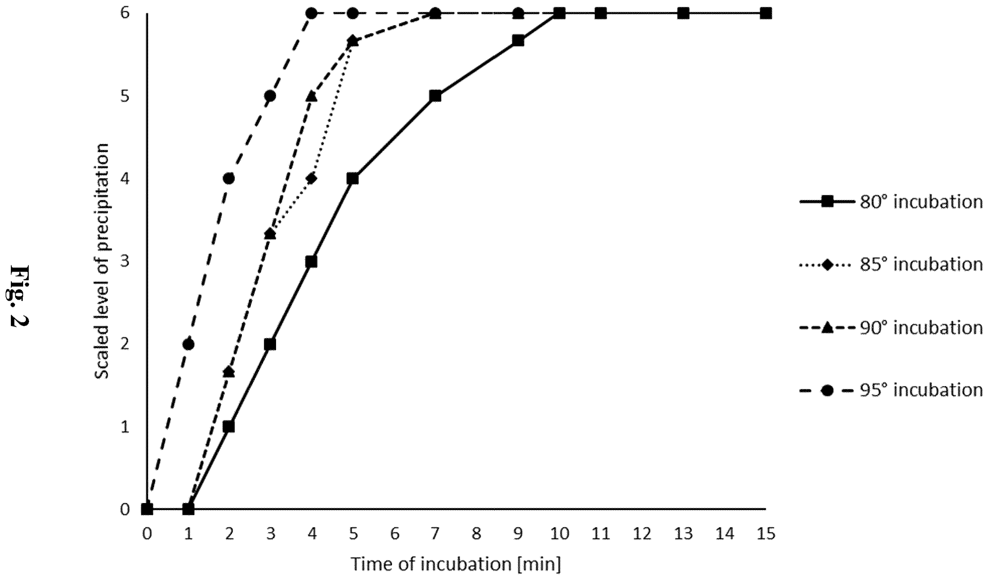

Fig. 2 illustrates the precipitation level over time for bovine serum albumin in TAP buffer at pH 5. The levels of precipitation have been visually determined at various time points and classified according to the following scheme (y-axis): 0: clear/ no precipitation; 1: weak/ starts getting cloudy; 2: medium/ slurry-like mixture; 3: slurry starts getting flaky, 4: very flaky mixture; 5: precipitate starts forming; 6: strong precipitation. At all temperatures, BSA showed eventually strong precipitation, with higher the temperatures leading to faster onset of strong precipitation.

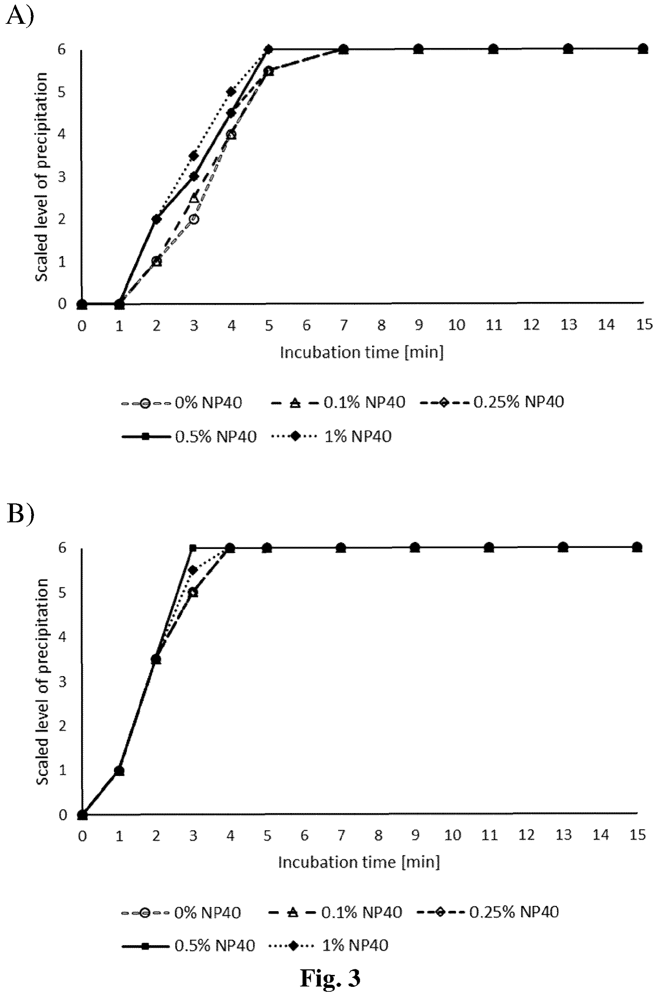

Fig. 3 illustrates, that ethoxylated nonylphenol (NP-40) may support precipitation of bovine serum albumin, with the effect being more prominent at lower temperatures and higher concentrations of NP-40. A) BSA precipitation over 15 minutes of incubation at 80°C; B) BSA precipitation over 15 minutes of incubation at 95°C. The levels of precipitation have been visually determined at various time points and classified according to the following scheme (y-axis): 0: clear/ no precipitation; 1: weak/ starts getting cloudy; 2: medium/ slurry-like mixture; 3: slurry starts getting flaky, 4: very flaky mixture; 5: precipitate starts forming; 6: strong precipitation.

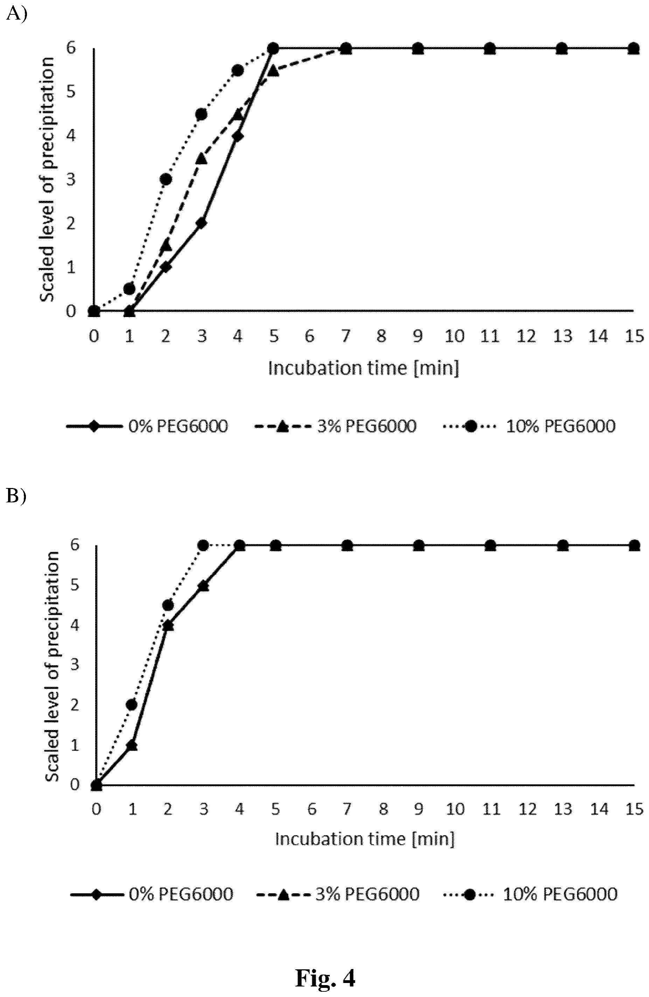

Fig. 4 shows the impact of polyethylene glycol (PEG 6000) (3% and 10% (w/v)) on precipitation of BSA in TAP buffer over time. A) BSA precipitation over 15 minutes of incubation at 80°C; B) BSA precipitation over 15 minutes of incubation at 95°C. The classification scheme for precipitation is as set out above for Fig.2. Thus, polyethylene glycol may support precipitation of bovine serum albumin, with the effect being more prominent at lower temperatures and higher concentrations of polyethylene glycol.

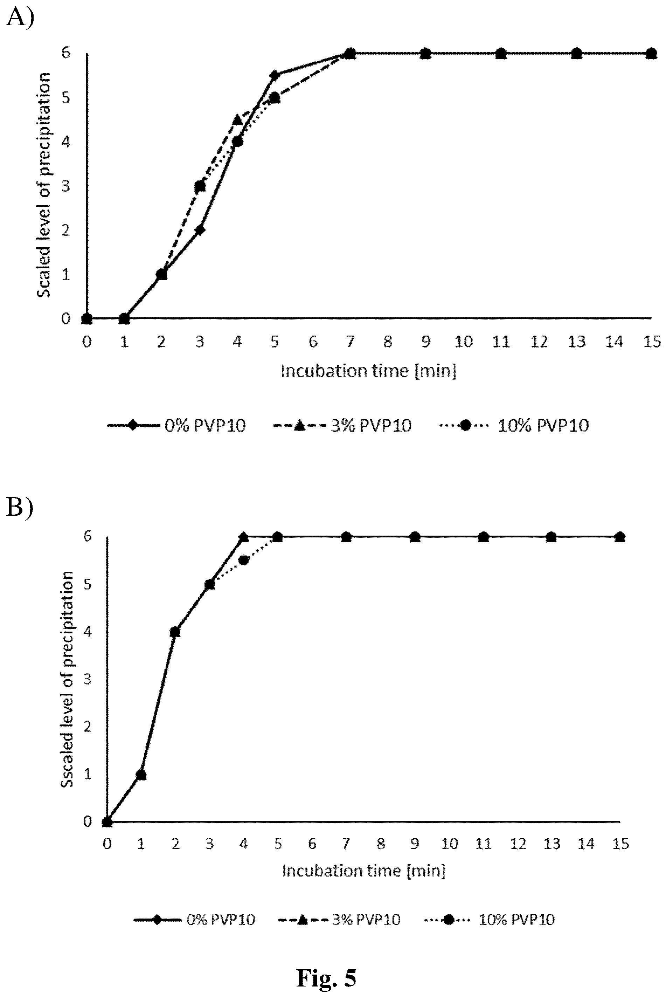

Fig. 5 shows the impact of polyvinylpyrrolidone (PVP-10) (3% and 10% (w/v)) on precipitation of BSA in TAP buffer over time. The classification scheme for precipitation is as set out above for Fig.2. A) BSA precipitation over 15 minutes of incubation at 80°C; B) BSA precipitation over 15 minutes of incubation at 95°C. Thus, polyvinylpyrrolidone may support precipitation of bovine serum albumin, with the effect being more prominent at lower temperatures, irrespective of the concentration of PCP-10.

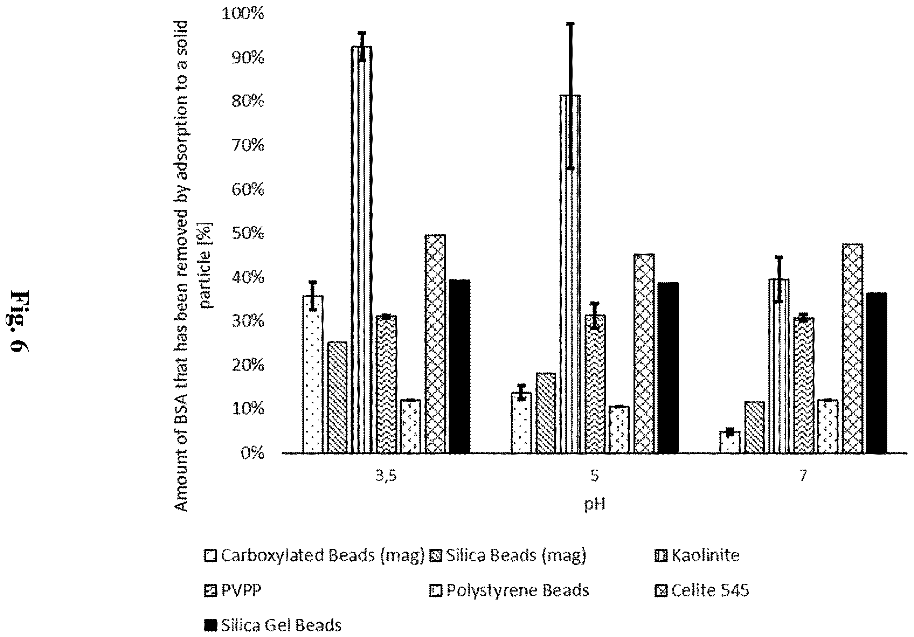

Fig. 6 illustrates the adsorption of bovine serum albumin to solid particles (magnetic and non-magnetic). The level of adsorption (%) was determined by measuring the absorbance at 280nm (A280) of the initial sample as well as of the supernatant after removal of the solid particles, either be magnetic separation (in case of magnetic particles) or by centrifugation (non-magnetic particles). Magnetic particles: Carboxylated beads, silica beads, polystyrene beads; Non-magnetic particles: kaolinite, polyvinylpolypyrollidone, Celite^{®} 545, silica beads. As a result, BSA partially adorbs to solid particles, which can thus facilitate separation of bovine serum albumin from a sample.

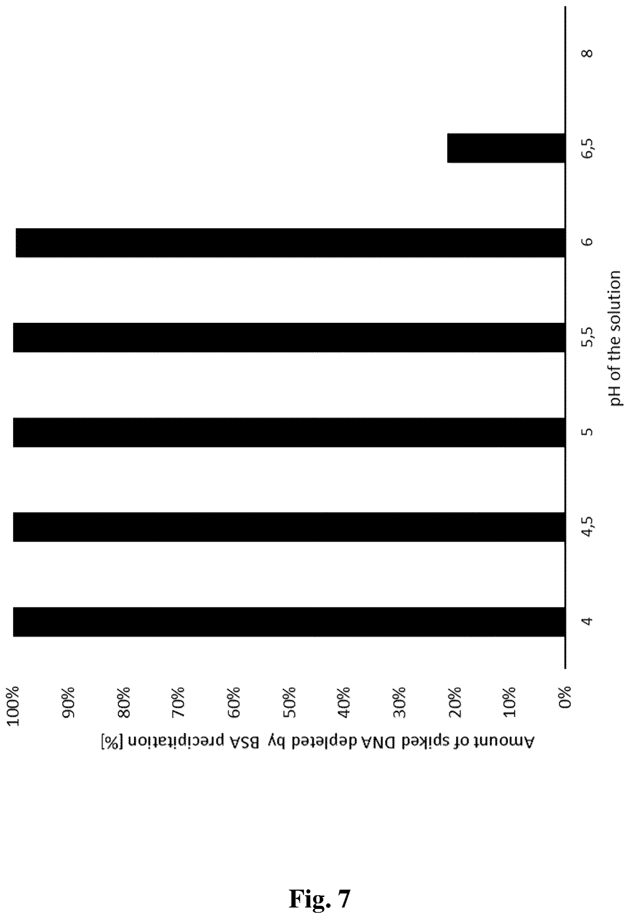

Fig. 7 illustrates the DNA depletion efficiency for BSA for various pH values in TAP buffer after 10 min of incubation at 90°C followed by 15 minutes at 4°C. The amount of depleted DNA was determined by measuring the DNA concentration by means of qPCR in the initial solutions before and after precipitation. As a result, BSA precipitates very efficiently at pH values in the range of 4.0 to 6.0.

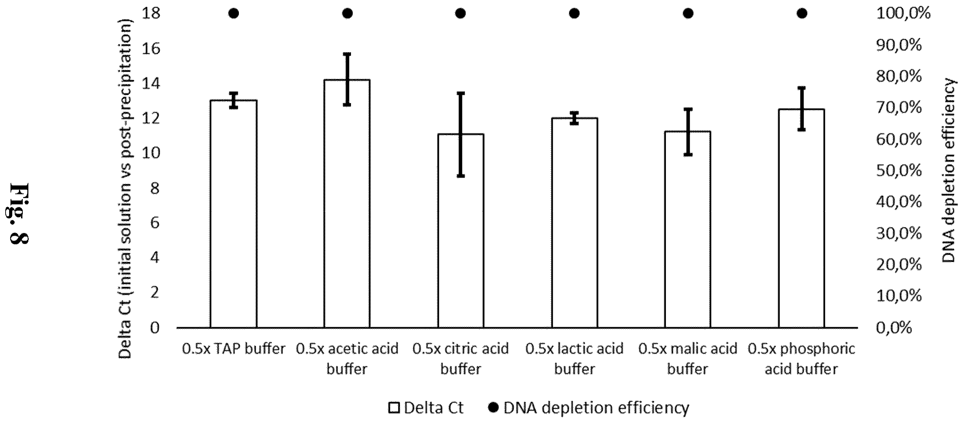

Fig. 8 illustrates the DNA depletion efficiency for BSA for various buffers at pH 5. The specific type of buffer has no substantial impact on DNA depletion efficiency.

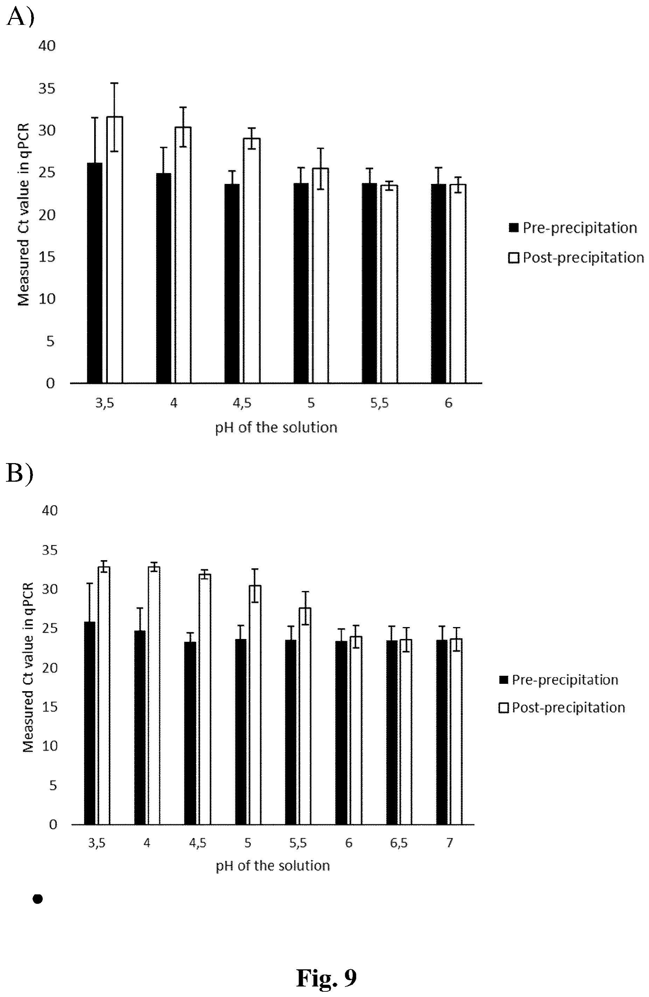

Fig. 9 illustrates the DNA depletion efficiency for lactalbumin and ovalbumin for various pH values in TAP buffer after 10 min of incubation at 90°C followed by 15 minutes at 4°C. A) lactalbumin B) ovalbumin.

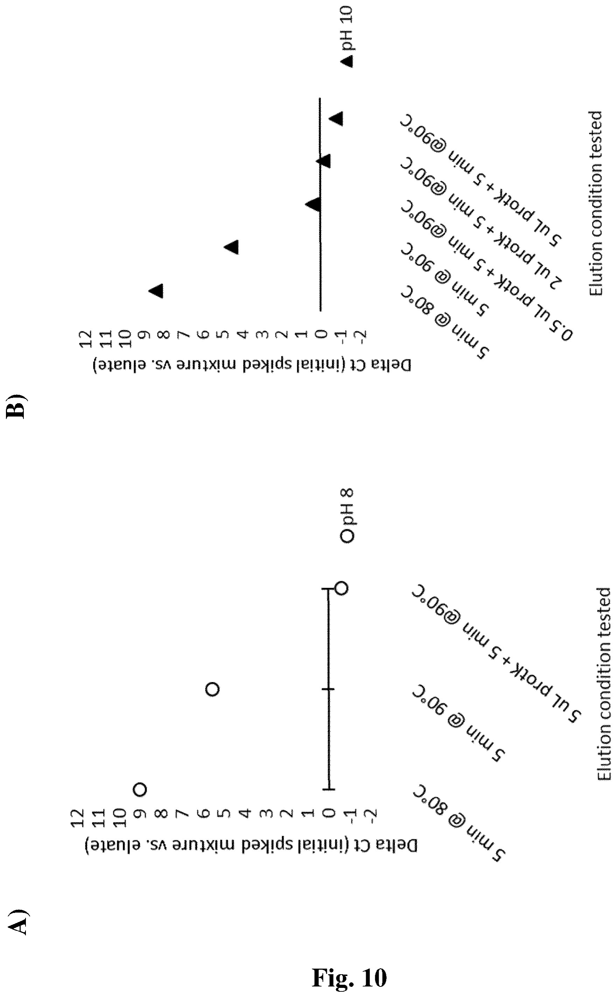

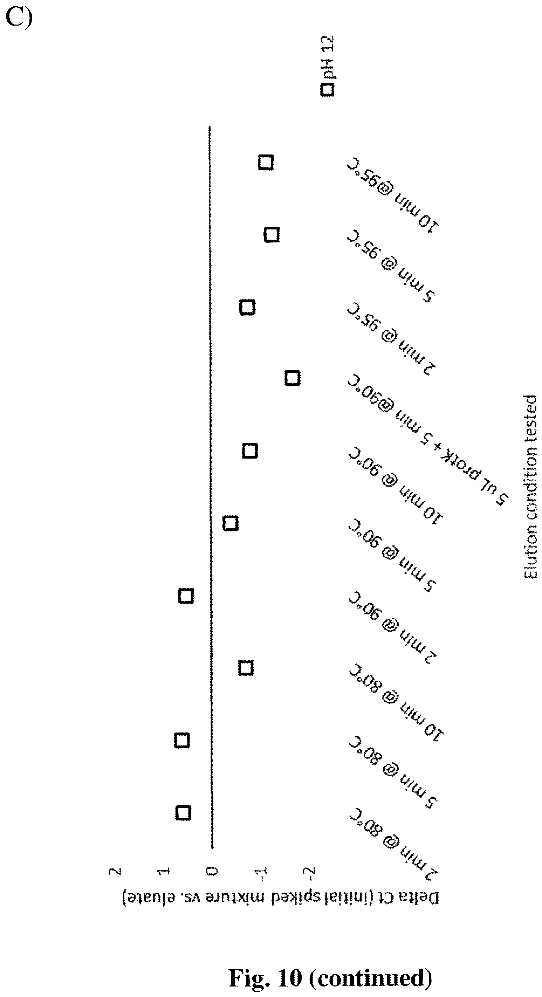

Fig. 10 illustrates the DNA elution efficiency under various elution conditions. (A) Elution efficiencies at pH 8 at different temperatures and incubation times as well as in the presence of different additives; (B) Elution efficiencies at pH 8 at different temperatures and incubation times as well as in the presence of different additives; (C) Elution efficiencies at pH 12 at different temperatures and incubation times as well as in the presence of different additives.

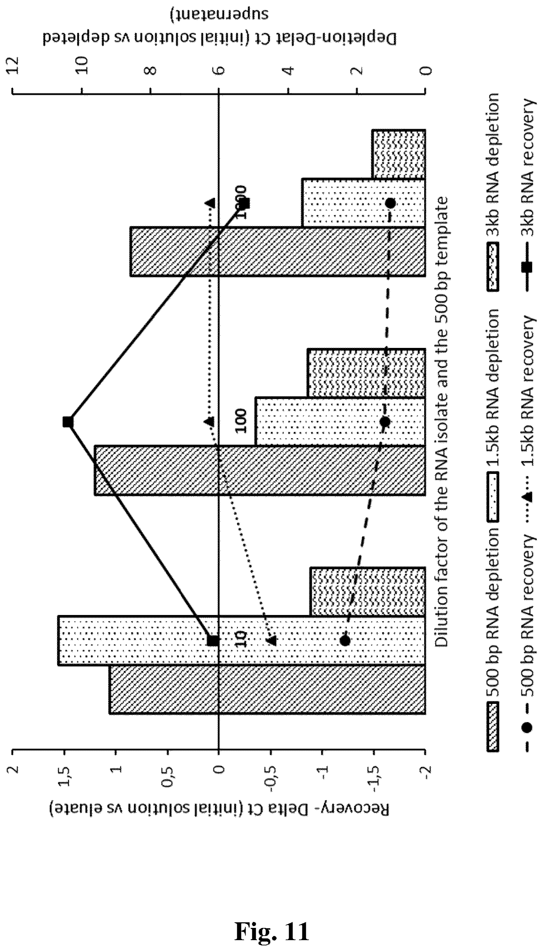

Fig. 11 illustrates depletion and recovery of RNA of different length and concentration with the method of the present invention. The bars are representing the extent of RNA depletion from the solution by precipitation of BSA. The lines are representing the concentration of RNA recovered after the extraction process in comparison to the concentration of RNA initially present in the sample.

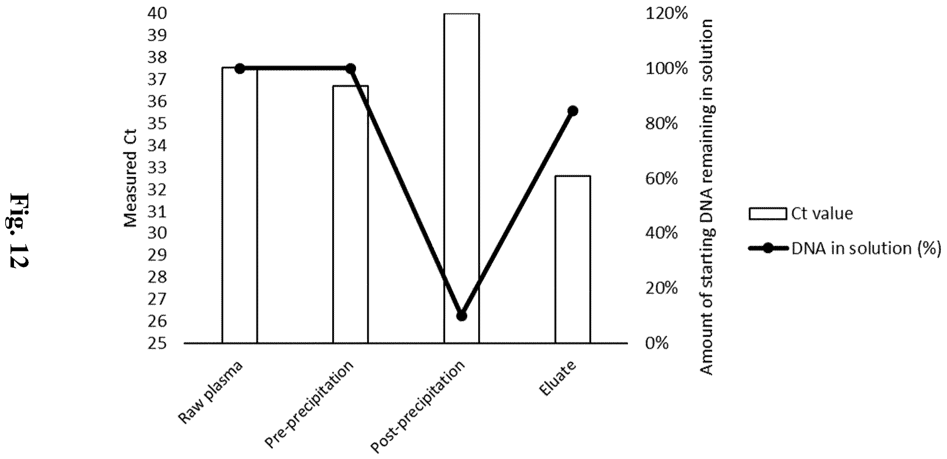

Fig. 12 illustrates the extraction of cell-free DNA from human plasma. The open bars represent the Ct values of the PCR-based quantification of samples taken throughout the extraction process. The filled circles represent the amount of human DNA in each liquid throughout the extraction process normalized to the amount of DNA present in the original sample.

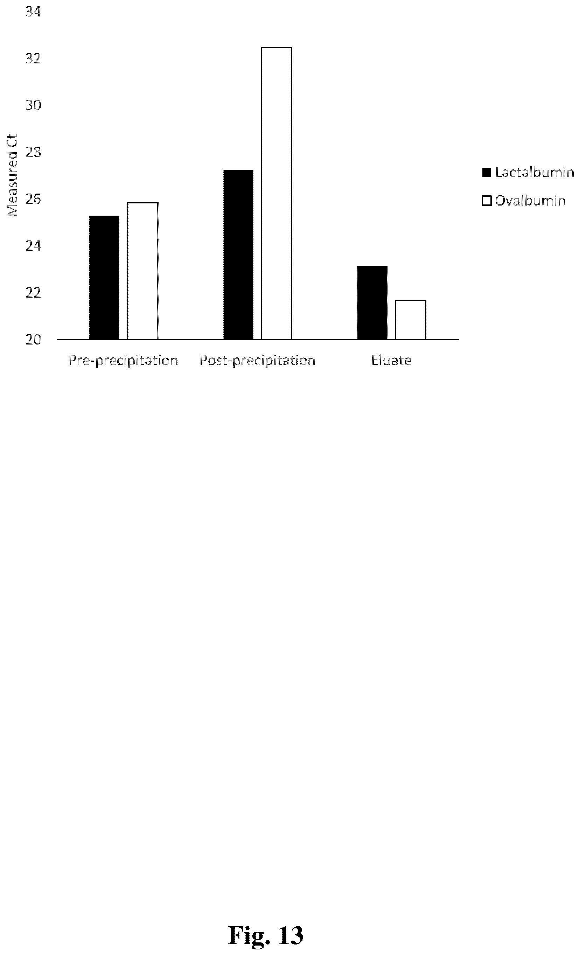

Fig. 13 shows that lactalbumin and ovalbumin can be obtained by the methods of the present invention. The graph shows that "eluate"-Cts < "pre-precipitation"-Cts < "post-precipitation-Cts. This indicates that the DNA is forming a complex with the precipitated albumin and the magnetic particles. After magnetic separation, the supernatant contains less DNA than before the precipitation. Furthermore, the DNA concentration in the solution after the elution step is higher than in the initial solution. This clearly indicated that the DNA is not only removed from the liquid during precipitation but can also be released from this complex.

### Examples

In the following, specific examples illustrating embodiments and aspects of the invention are presented. However, the present invention shall not to be limited in scope by the specific examples described herein. Indeed, various modifications of the invention in addition to those described herein will become readily apparent to those skilled in the art from the foregoing description and the examples below. All such modifications fall within the scope of the appended claims.

### Example 1: Protein precipitation scan for four different albumins over broad pH range

In order to study the precipitation of different albumins the following materials were used: bovine serum albumin (BSA), ovalbumin, lactalbumin, human plasma, TAP buffer (50 mM acetate, 50 mM phosphate, 50 mM Tris, 25 mM NaCl) adjusted to pH 3.5/ 4/ 4.5/ 5/ 5.5/ 6/ 6.5/ 7/ 7.5/ 8/ 9, respectively, and distilled water.

For each albumin (BSA, lactalbumin, ovalbumin), a 4% (m/v) solution in distilled water was prepared. 62.5 µL of each of these 4% albumin solutions were then added to 437.5 µL of each pH-adjusted TAP buffer (50 mM acetate, 50 mM phosphate, 50 mM Tris, 25 mM NaCl). The samples were mixed and spun down shortly to remove any material from the lid. Subsequently, absorbance at 280 nm of these pre-precipitation solution samples was determined using a spectrophotometer. The solutions were then incubated at 90°C for 5 minutes and centrifuged thereafter for 3 minutes to settle the precipitated albumin. Thereafter, absorbance of the supernatant of the samples at 280 nm was determined again and the difference in albumin concentration between the respective pre- and post-precipitation sample was calculated based on the absorbance values obtained for each sample. Human plasma, which comprises human serum albumin, was in principle processed in analogous manner. However, no solution was prepared in the first step. Instead, 62.5 µL of human plasma (assumed to have an HSA concentration of approx. 4%) were directly added to 437.5 µL of each pH-adjusted TAP buffer vials.

As a result, all four albumins (BSA, lactalbumin, ovalbumin and human serum albumin) show good precipitation at low pH, in particular in the range of pH 4 to pH 6 be precipitated in a pH range. However, for lactalbumin, the extent of precipitation showed a much less stringent dependence on the pH compared to the other albumins tested.

### Example 2: Protein precipitation scan for bovine serum albumin over time

To assess protein precipitation of bovine serum albumin over time, seven BSA containing samples were prepared. Each time, 62.5 µL of a 4% (m/v) solution of BSA in distilled water were added to 437.5 µL of TAP buffer (pH 5). The samples were mixed and spun down shortly to remove any material from the lid. The seven samples were then incubated for 15 minutes, each at a different temperature: 20°C/ 40°C/ 60°C/ 80°C/ 85°C/ 90°C/ 95°C. In 1- minute intervals, the degree of precipitation in each of the sample was visually determined. The following classification was used: 0: clear / no precipitation; 1: weak/starts getting cloudy; 2: medium/slurry-like mixture; 3: slurry starts getting flaky; 4: very flaky mixture; 5: precipitate starts forming; 6: strong precipitation.

As a result, all samples heated to temperatures above 60°C reached within 5 minutes the status of a very flaky mixture and within 10 minutes all these samples showed strong precipitation. Overall, higher temperatures correlated with earlier onset of precipitation.

### Example 3: Protein precipitation scan for bovine serum albumin in presence of octylphenoxy poly(ethyleneoxy)ethanol

In order to assess the impact of octylphenoxy poly(ethyleneoxy)ethanol IGEPAL^{®} CA-630 (Sigma-Aldrich) on precipitation, 10 BSA containing samples were prepared. Each time, 62.5 µL of a 4% (m/v) solution of BSA in distilled water were added to 437.5 µL of TAP buffer (pH 5), with two of the samples (pairs) always having the same concentration of octylphenoxy poly(ethyleneoxy)ethanol (0%/ 0.1%/ 0.25%/ 0.5%/ 1% (v/v)). The samples were mixed and spun down shortly to remove any material from the lid. The samples were then incubated for 15 minutes, with the members of a pair (same concentration in octylphenoxy poly(ethyleneoxy)ethanol) incubated at a different temperature: 80°C / 95°C. In 1- minute intervals, the degree of precipitation in each of the sample was visually determined. The classification of example 2 was used to classify the level of precipitation.

As a result, octylphenoxy poly(ethyleneoxy)ethanol) increased the BSA precipitation rate at 80°C, whereas at 95°C, this effect was negligible.

### Example 4: Protein precipitation scan for bovine serum albumin in presence of polyethylene glycol

In order to assess the impact of polyethylene glycol (PEG) on precipitation, 6 BSA containing samples were prepared. Each time, 62.5 µL of a 4% (m/v) solution of BSA in distilled water were added to 437.5 µL of TAP buffer (pH 5), with two of the samples (pairs) always having the same concentration of polyethylene glycol (PEG-6000, Sigma Aldrich), namely either 0%, 3% or 10% (m/v) of PEG. The samples were mixed and spun down shortly to remove any material from the lid. The samples were then incubated for 15 minutes, with the members of a pair (same concentration in PEG) incubated at a different temperature, namely either 80°C or 95°C. In 1- minute intervals, the degree of precipitation in each of the sample was visually determined. The classification of example 2 was used to classify the level of precipitation and mean values were determined.

As a result, PEG increased the BSA precipitation rate at 80°C as well as at 95°C as compared to no PEG. Furthermore, the higher PEG concentration also showed better BSA precipitation than the lower PEG concentration at both temperatures.

### Example 5: Protein precipitation scan for bovine serum albumin in presence of polyvinylpyrrolidone

In order to assess the impact of polyvinylpyrrolidone (PVP) on precipitation, 6 BSA containing samples were prepared. Each time, 62.5 µL of a 4% (m/v) solution of BSA in distilled water were added to 437.5 µL of TAP buffer (pH 5), with two of the samples (pairs) always having the same concentration of polyvinylpyrrolidone (PVP-10, Sigma Aldrich, having an average molecular weight of 10,000), namely either 0%, 3% or 10% (m/v) of PVP. The samples were mixed and spun down shortly to remove any material from the lid. The samples were then incubated for 15 minutes, with the members of a pair (same concentration in PVP) incubated at a different temperature, namely either 80°C or 95°C. In 1- minute intervals, the degree of precipitation in each of the sample was visually determined. The classification system of example 2 was used to classify the level of precipitation and mean values were determined.

As a result, PVP slightly increased the BSA precipitation rate at 80°C as compared to no PVP. In contrast, at 95°C no significant effect of the addition of PVP have been observed.

### Example 6: Adsorption of bovine serum albumin to solid particles

In order to determine the adsorption rate of bovine serum albumin to solid particles, seven different types of solid particles were used (magnetic where indicated): carboxylates beads (magnetic), silica beads (magnetic), polystyrene beads (magnetic), kaolinite, polyvinylpolypyrrolidone (PVPP), Celite^{®} 545 (Sigma Aldrich), and silica gel beads.

For experiments with the magnetic particles, 4 tubes containing 60 µL carboxylated beads, 4 tubes containing 60 µL magnetic silica beads, and 4 tubes containing 60 µL magnetic polystyrene beads were prepared. The tubes containing the beads were placed on a magnetic rack for 30 seconds to allow for separation of the particles from the liquid. The liquid supernatant was then discarded and the magnetic beads were resuspended in 200 µL of 0.25% (m/v) solution of BSA in TAP buffer adjusted to pH 3.5/ 5/ 7/ 9. The tubes were then incubated for 5 minutes at room temperature and again placed on a magnetic rack allowing the particles some time to separate. The supernatant was then collected and the absorbance at 280 nm measured using a spectrophotometer. The difference between the A₂₈₀ of the supernatant and the respective initial BSA solution in TAP buffer was then calculated to determine how much BSA had been co-precipitated with the magnetic particles due to adsorption of BSA to such solid particle.

For experiments with the non-magnetic particles, 4 tubes containing 50 mg Kaolinite, 4 tubes containing 50 mg PVPP, 4 tubes containing 50 mg Celite^{®} 545, and 4 tubes containing 50 mg silica gel beads were prepared. 500 µL distilled water where then added to each tube and the tubes thoroughly mixed to wash the particles. Afterwards, the samples were centrifuged for 1 minute at 5000 x g to settle the solid particles. The liquid supernatant was then discarded and the beads were resuspended in 200 µL of 0.25% (m/v) solution of BSA in TAP buffer adjusted to pH 3.5/ 5/ 7/ 9. The tubes were then incubated for 5 minutes at room temperature and again centrifuged for 5 min at 5000 x g. The supernatant was then collected and the absorbance at 280 nm measured using a spectrophotometer. The difference between the A₂₈₀ of the supernatant and the respective initial BSA solution in TAP buffer was then calculated to determine how much BSA had been co-precipitated with the non-magnetic particles due to adsorption of BSA to such solid particle.

As a result, BSA adsorption varies with the type of bead used and pH, but never reached 100%. Adsorption at lower pH values (3.5 and 5) was in most cases more prominent than at high pH values (7 and 9).

### Example 7: Efficiency of BSA-facilitated DNA depletion at different pH values

In order to assess the efficiency of DNA depletion from a sample by way of albumin precipitation, seven BSA containing samples were prepared, each sample having a different pH. 62.5 µL of 4% BSA solution in distilled water and 20 µL human genomic DNA extracted previously from a blood sample were added to 417.5 µL of each TAP buffer (50 mM acetate, 50 mM phosphate, 50 mM Tris, 25 mM NaCl), adjusted to pH 4, 4.5, 5, 5.5, 6, 6.5, or 8, respectively. The samples were mixed and spun down shortly to remove any material from the lid. 5 µL of each solution were collected for later use as pre-precipitation controls. The samples were then incubated for 10 minutes at 90°C and then cooled down for 15 minutes at 4°C. Subsequently, the samples were centrifuged for 3 minutes at 8000 x g and the supernatant collected. In a next step, the pre-precipitation solutions and the collected final supernatants were analysed by means of qPCR and the difference in Ct values calculated to evaluate the amount of DNA depleted from the solution via co-precipitation with BSA.As a result, the genomic DNA was efficiently depleted (100%) in the pH range of 4 to 6, and a partial depletion was still observed at a pH of 6.5, while no depletion occurred at a pH of 8.

### Example 8: The impact of the type of buffer on DNA depletion efficiency

In order to assess the impact of the buffer on DNA depletion, six different buffer systems were testes at pH5. For this purpose, 62.5 µL of 4% BSA solution in distilled water and 2 µL of genomic DNA (Cambio) were added to 425.5 µL of six different buffers adjusted to pH 5. The following buffers were used: TAP buffer (50 mM acetate, 50 mM phosphate, 50 mM Tris, 25 mM NaCl, pH 5), 50mM acetate buffer (pH 5), 50mM citric acid buffer (pH 5), 50mM lactic acid buffer (pH 5), 50mM malic acid buffer (pH 5) and 50mM phosphate buffer (pH 5). The samples were mixed and spun down shortly to remove any material from the lid. Afterwards, 5 µL of each solution were collected for later use as pre-precipitation controls. Next, 10 µL of carboxylated beads were added to each of the mixtures and the samples were mixed again. The samples were then incubated for 10 minutes at 90°C and subsequently cooled down for 15 minutes at 4°C. Thereafter, the samples were placed on a magnetic stand at room temperature and the beads allowed for 1 minute to separate from the sample. Subsequently, the supernatant was collected. In a next step, the pre-precipitation solutions and the collected final supernatants were analysed by means of qPCR and the difference in Ct values calculated to evaluate the amount of DNA depleted from the solutions via co-precipitation with BSA.

As a result, all buffer systems performed equally well and achieved the same DNA depletion efficiency (100%).

### Example 9: Efficiency of lactalbumin- or ovalbumin-facilitated DNA depletion at different pH values

In order to assess the DNA depletion efficiency with other albumins (lactalbumin and ovalbumin), various albumin containing samples adjusted to different pH values (pH 3.5, 4.0, 4.5, 5.0, 5.5 and 6.0 for lactalbumin and om the range of 3.5, 4.0, 4.5, 5.0, 5.5, 6.5 and 7.0 for ovalbumin) were prepared. Briefly, 4% (m/v) solutions with lactalbumin or ovalbumin, respectively, in distilled water was prepared. 62.5 µL of these 4% albumin solutions and 20 µL of human genomic DNA were then added to 417.5 µL of each pH-adjusted TAP buffer. The samples were mixed and spun down shortly to remove any material from the lid. Afterwards, 5 µL of each solution were collected for later use as pre-precipitation controls. The samples were then incubated for 10 minutes at 90°C and subsequently cooled down for 15 minutes at 4°C. Thereafter, the samples were centrifuged for 3 minutes at 8000 x g and the supernatant was collected. In a next step, the pre-precipitation solutions and the collected final supernatants were analysed by means of qPCR and the difference in Ct values calculated to evaluate the amount of DNA depleted from the solutions via co-precipitation with the respective albumin.

As a result, lactalbumin as well as ovalbumin depleted the DNA efficiently over a pH range that is within the range of good precipitation behaviour (see results of experiment 1). Therefore, albumins in general can be used to deplete nucleic acids from a sample.

### Example 10: DNA elution efficiency of different elution conditions

In an attempt to determine the elution efficiency of DNA from precipitated albumin the following experiment was carried out. Various 4% (m/v) solution of BSA in distilled water was prepared. 62.5 µL of this 4% BSA solution and 2 µL of genomic DNA were added to 425.5 µL TAP buffer at pH 5. The samples were mixed and spun down shortly to remove any material from the lid. 5 µl of each solution were collected for later use as pre-precipitation controls. The sample were then incubated for 10 minutes at 80°C and then cooled down for 15 minutes at 4°C. Thereafter, 30 µL of carboxylated beads were added to each of the solutions and the solutions mixed. Next, the solutions were placed on a magnetic rack at room temperature and the beads allowed to separate from the sample for 1 minute. The supernatant was then discarded and the bead pellet resuspended in 400 µL of elution buffer (see below). The samples were again mixed and spun down shortly to remove any remaining material form the lid of the tube. Next, the samples were incubated for the indicated times at the indicated temperatures (see below). Thereafter, the solutions were placed again on a magnetic rack at room temperature and the beads allowed to separate from the sample for 1 minute and the supernatant collected. The following elution conditions were tested:
∘ Beads resuspended in 400 µL TAP buffer adjusted to pH 8 and elution was carried out i) at 80°C for 5 minutes, ii) at 90°C for 5 minutes and iii) at 90°C for 5 minutes with additionally 5 µL proteinase K added.
∘ Beads resuspended in 400 µL TAP buffer adjusted to pH 10 and elution was carried out i) at 80°C for 5 minutes, ii) at 90°C for 5 minutes and iii) at 90°C for 5 minutes with additionally 0.5 µL proteinase K added, iv) at 90°C for 5 minutes with additionally 2 µL proteinase K added and v) at 90°C for 5 minutes with additionally 5 µL proteinase K added.
∘ Beads resuspended in 400 µL TAP buffer adjusted to pH 12 and elution was carried out i) at 80°C for 2 minutes, ii) at 80°C for 5 minutes, iii) at 80°C for 10 minutes, iv) at 90°C for 2 minutes, v) at 90°C for 5 minutes, vi) at 90°C for 10 minutes, vii) at 90°C for 5 minutes with additionally 5 µL proteinase K added, viii) at 95°C for 2 minutes, ix) at 95°C for 5 minutes, and x) at 95°C for 10 minutes.

As a result, the DNA elution at pH 8 and pH 10 is moderately effective with improved results in the presence of proteinase K. At pH 12, excellent elution does not require presence of proteinase K. Instead, the elution seems to be time dependent but not temperature dependent.

### Example 11: RNA extraction

For extraction of RNA, the following experiment was carried out: Dilutions (1:10, 1:100 and 1:1000) of *S. aureus* ribosomal RNA isolate (containing the two ribosomal RNAs of 1.5 kb and 3 kb) and of a 500 bp artificial RNA template, respectively, in distilled water were prepared. Each of the dilutions contained all three types of RNA. In addition, a 4% (m/v) solution of BSA in distilled water was prepared. Each dilution was processed as follows: In a tube, 425.5 µl TAP buffer (pH 5) and 62.5 µL of the 4% BSA solution and 2 µL RNA dilution were added. 5 µL of each of these solutions were collected and served as pre-precipitation controls. The tubes were then incubated for 10 minutes at 80°C and immediately thereafter cooled down for 5 minutes at 4°C. Thereafter, 10µl of carboxylated beads where added to each of the mixtures and mixed will by vortexing. The tubes were placed for 1 minute on a magnetic stand at room temperature to allow separation of the beads. Thereafter, the supernatant was discarded and the bead pellet resuspend in 400 µl of TAP buffer (pH 10). In a next step, 2 µL of proteinase **K** were added to each mixture, the samples were thoroughly vortexed and briefly spun down to remove any remaining material from the lid of the tube. The tubes were then incubated at 90°C for 5 minutes. Afterwards, the tubes were again placed on a magnetic rack for 1 minute at room temperature to allow separation of the beads. Afterwards, the supernatant was collected and an RT-qPCR performed for all of the initial pre-precipitation solutions, the RNA-depleted supernatants after the heat-based BSA precipitation and the final eluates. Finally, the difference in Ct values were calculated to evaluate the amount of RNA (detection for 3 different sized RNA sequences: 500 bp of the artificial RNA sequence, 1.5 kb and 3 kb of the S. *aureus* ribosomal RNA isolate) depleted from the solution via co-precipitation with albumin and the final amount of RNA re-eluted into the elution buffer.

As a result, RNAs of a length up to 1.5 kb are completely depleted by BSA precipitation throughout the tested concentration range. In contrast, the 3 kb RNA showed a depletion efficiency of ca. 90% at the highest test concentration to ca. 30% at the lowest test concentration. The elution and recovery of the RNAs was slightly more efficient for shorter species. Yet, the elution worked similarly well over all RNA concentrations.

### Example 12: Extraction of DNA from human plasma

In order to test the extraction of DNA from a biological sample already containing albumin, 300 µL of human venous blood were collected in EDTA tubes and centrifuged for 10 minutes at 2000 x g. 100 µL of the plasma on the top layer were transferred to a new tube and 100 µL of TAP buffer adjusted to pH 5, 790 µL distilled water and 10 µL of carboxylated beads were added. The samples were mixed and 20 µL were collected as as a pre-precipitation control. The samples were then incubated at 90°C for 5 minutes. Afterwards, the samples were briefly mixed, quickly spun down and placed on a magnetic rack for one minute to allow magnetic separation of the beads. In a next step, the supernatant was removed and the bead pellet resuspended in 200 µL of TAP buffer adjusted to pH 12. The samples were incubated for 5 minutes at 90°C and again briefly mixed, quickly spun down and placed on a magnetic rack for one minute to allow magnetic separation of the beads. Finally, the eluate was collected.

As a result, human cell-free DNA was successfully extracted from the blood plasma sample with a recovery rate ca. 85% in the final eluate.

### Example 13: Extraction and elution of DNA with lactalbumin and ovalbumin

62.5 µL of lactalbumin/ ovalbumin solution (4% in H₂O) was added to 935.5 µL TAP buffer adjusted to pH 5. To that, female genomic placental DNA was added. The mixture was mixed well and 20 µL were collected as "pre-precipitation" control (sample 1). To the solution, 10 µL Beads A were added, the mixture was mixed well via vortexing and incubated for 5 minutes at 90°C. The tube was placed on a magnetic rack and allowed 2 minutes for magnetic separation of the beads. The supernatant was collected as "post-precipitation" control (sample 2). The beads were resuspended in 400 µL TAP buffer adjusted to pH 12, mixed well and incubated for 5 minutes at 90°C. The tube was then placed on a magnetic rack and 2 minutes were allowed for magnetic separation. The "eluate" was collected (sample 3).

The DNA concentration in all three samples from each albumin type were quantified using real-time PCR.

## Claims

1. Method for isolating nucleic acids, the method comprising the following steps:
a) providing a sample comprising nucleic acids, a buffer and soluble albumin;
b) precipitating the albumin in the sample; and
c) separating the precipitated albumin from the supernatant of the sample,
wherein the sample of step a) has a pH of 6.5 or lower, preferably in the range of 3.5 to 6.5.

2. The method according to claim 1, wherein the sample of step a) has a pH in the range of 4.0 to 6.0

3. The method according to claim 1, wherein the sample of step a) has a pH in the range of - 1.2 pH to +1.2 pH compared to the isoelectric point (IEP) of the albumin.

4. The method according to any one of claims 1 to 3, wherein the soluble albumin is present in step a) in a concentration in the range of 0.05% to 0.5 % (w/v).

5. The method according to any one of the preceding claims, wherein the soluble albumin is selected from bovine serum albumin (BSA), human serum albumin, ovalbumin and lactalbumin, preferably wherein the soluble albumin is bovine serum albumin (BSA).

6. The method according to claim 5, wherein the soluble albumin is BSA and wherein the sample of step a) comprises a pH in the range of 4.5 to 5.5.

7. The method according to any one of the preceding claims, wherein precipitating in step b) involves incubating the sample at a temperature of at least 70°C, preferably at least 80°C, for a sufficient amount of time to precipitate the soluble albumin.

8. The method according to any one of the preceding claims, wherein the sample of step a) comprises additionally magnetic or non-magnetic particles.

9. The method according to any one of claims 1 to 7, wherein the method comprises the additional step of:
b') mixing the albumin precipitated in step b) with magnetic or non-magnetic particles.

10. The method according to claim 8 or claim 9, wherein the particles are magnetic particles.

11. The method according to any one of the preceding claims, wherein the precipitate comprises albumin and magnetic particles and wherein the precipitate is separated from the supernatant of the sample using a magnet.

12. The method according to any one of the preceding claims, wherein the method comprises the additional step of:
d) eluting the nucleic acids from the precipitated albumin by incubating the precipitated albumin in an elution buffer having a pH in the range of 8 to 12.

13. The method according to claim 12, wherein the elution buffer comprises a protease such as proteinase K and/or wherein the precipitated albumin is incubated in the elution buffer at a temperature in the range of 85°C to 95°C.

14. Composition or kit comprising i) a buffer suitable for buffering a sample at a pH of 6.5 or lower, preferably in the range of 3.5 to 6.5, more preferably at a pH of 5, b) soluble albumin and c) magnetic or non-magnetic particles.

15. The kit according to claim 14, wherein the kit additionally comprises an elution buffer which provides for a pH in the range of 8 to 12, preferably in the range of 10 to 12.
